(19) Europäisches Patentamt
European Patent Office
Office européen des brevets

(11) **EP 1 801 233 B1**

(12) **EUROPÄISCHE PATENTSCHRIFT**

(45) Veröffentlichungstag und Bekanntmachung des Hinweises auf die Patenterteilung:
**21.10.2009 Patentblatt 2009/43**

(51) Int Cl.:
*C12Q 1/37* [(2006.01)]    *G01N 33/573* [(2006.01)]

(21) Anmeldenummer: **06024985.1**

(22) Anmeldetag: **04.12.2006**

(54) **Diagnostisches Verfahren zur Erkennung von Trägern der Marburg I-Variante der Faktor VII-aktivierenden Protease (FSAP) anhand differenzieller Modulation der FSAP-Aktivität**

Method of diagnosis for recognition of carriers of the Marburg I variant of factor VII activating protease (FSAP) by differential modulation of FSAP activity

Procédure diagnostique de reconnaissance des porteurs de la variante de Marburg I de la protéase activatrice du facteur VII (FSAP) au moyen de la modulation différentielle de l'activité de la FSAP

(84) Benannte Vertragsstaaten:
**AT BE BG CH CY CZ DE DK EE ES FI FR GB GR HU IE IS IT LI LT LU LV MC NL PL PT RO SE SI SK TR**

(30) Priorität: **22.12.2005  DE 102005062055**
**22.12.2005  DE 102005062053**

(43) Veröffentlichungstag der Anmeldung:
**27.06.2007 Patentblatt 2007/26**

(73) Patentinhaber: **Siemens Healthcare Diagnostics Products GmbH**
**35041 Marburg (DE)**

(72) Erfinder:
• **Vitzthum, Frank, Dr.**
**35094 Lahntal-Sterzhausen (DE)**
• **Schwarz, Herbert**
**35102 Lohra (DE)**

(56) Entgegenhaltungen:
**US-A1- 2002 142 316    US-A1- 2003 215 447**

• **ROEMISCH J ET AL: "THE FREQUENT MARBURG I POLYMORPHISM IMPAIRS THE PRO-UROKINASE ACTIVATING POTENCY OF THE FACTOR VII ACTIVATING PROTEASE (FSAP)" BLOOD COAGULATION & FIBRINOLYSIS, RAPID COMMUNICATIONS, OXFORD,OXFORD, GB, Bd. 13, Nr. 5, Juli 2002 (2002-07), Seiten 433-441, XP009013813 ISSN: 0957-5235**
• **ROEMISCH J: "FACTOR VII ACTIVATING PROTEASE (FSAP): A NOVEL PROTEASE IN HEMOSTATIS" BIOLOGICAL CHEMISTRY, Bd. 383, Nr. 7/8, Juli 2002 (2002-07), Seiten 1119-1124, XP009013814 ISSN: 1431-6730**
• **ROEMISCH J ET AL: "QUANTIATION OF THE FACTOR VII- AND SINGLE-CHAIN PLASMINOGEN ACTIVATOR-ACTIVATING PROTEASE IN PLASMAS OF HEALTHY SUBJECTS" BLOOD COAGULATION & FIBRINOLYSIS, RAPID COMMUNICATIONS, OXFORD,OXFORD, GB, Bd. 12, Nr. 5, Juli 2001 (2001-07), Seiten 375-383, XP009013812 ISSN: 0957-5235**

**Beschreibung**

Die Erfindung liegt auf dem Gebiet der Diagnose der Marburg I (MR I)-Mutation der den Blutgerinnungsfaktor VII-aktivierenden Protease (FSAP), die bei ungefähr 5 bis 10 % der westeuropäischen Bevölkerung gefunden wird. Entsprechend einer Studie von Willeit et al. weisen heterozygote Träger der FSAP-MR I-Mutation gegenüber dem Durchschnitt der Bevölkerung ein höheres Risiko auf, eine Carotis Stenose zu entwickeln [Willeit et al. (2003): Marburg I polymorphism of factor VIIactivating protease: a prominent risk predictor of carotid stenosis. Circulation 107, 667-670]. Da das Vorliegen einer FSAP-MR I-Mutation einen potenziellen Marker für eine genetische Disposition für die Entwicklung atherosklerotischer Erkrankungen darstellt, ist die zuverlässige Erkennung von homo- oder heterozygoten Trägern der FSAP-MR I-Mutation besonders im Hinblick auf eine individuelle Gesundheitsvorsorge von Interesse.

Die FSAP ist eine plasmatische Serinprotease, die auch unter dem Namen PHBSP (plasma hyaluronan binding serine protease) beschrieben wurde. Die FSAP liegt im Humanplasma in einer Konzentration von ca. 12 µg/ml vor und kann über Autokatalyse vom einkettigen Proenzym (single chain-, sc-FSAP) in die aktive zweikettige Protease (two chain-, tc-FSAP) überführt werden. Die aktive Protease verfügt über verschiedene Funktionen bzw. Aktivitäten. Es ist bekannt, dass die FSAP einerseits die Fähigkeit besitzt, den Blutgerinnungsfaktor VII sowie Einketten-Plasminogenaktivatoren, wie Plasminogen-aktivierende Prourokinasen, wie scuPA (single chain urokinase plasminogen activator) und sctPA (single chain tissue plasminogen activator) zu aktivieren. Andererseits verfügt die FSAP über die Fähigkeit, die Blutgerinnungsfaktoren V/Va und VIII/VIIIa zu inaktivieren.

Verschiedene Testverfahren zur qualitativen oder quantitativen Bestimmung der FSAP, die sich diese biologischen Aktivitäten der FSAP zu Nutze machen, sind in EP 952 215 A2 beschrieben. Eine weitere Aktivität der FSAP, die eine Bestimmung der aktiven Protease ermöglicht und ebenfalls in vorgenanntem Patentdokument beschrieben ist sowie in Römisch et al. (1999): The FVII activating protease cleaves single-chain plasminogen activators. Haemostasis 29, 292-299 und in Hunfeld et al. (1999): Detection of a novel plasma serine protease during purification of vitamin K-dependent coagulation factors. FEBS Letters 456, 290-294, ist die amidolytische Aktivität der FSAP gegenüber niedermolekularen Substraten, insbesondere gegenüber dem chromogenen Substrat S-2288™ (HD-Ile-Pro-Arg-pNA).

Neben der Wildtypsequenz des humanen FSAP-Gens sind verschiedene Nukleotidpolymorphismen bekannt, die in zwei Fällen auch zu einer Änderung der Aminosäuresequenz führen (EP 1 182 258 A1). Die sogenannte Marburg I-Mutation (auch -Polymorphismus, -Allel oder -Variante) führt zu einem Gly/Glu Aminosäureaustausch an Position 534 des Proenzyms einschließlich des Signalpeptids (Gly/Glu 534) und resultiert in einer 50-80 %igen Verringerung der Prourokinase-aktivierenden Aktivität, während die Fähigkeit Faktor VII zu aktivieren unverändert erhalten bleibt. Eine weitere Mutation, die sogenannte Marburg II (MR II)-Mutation (auch MR II-Polymorphismus, -Allel oder -Variante) führt zu einem Glu/Gln Aminosäureaustausch an Position 370 des Proenzyms einschließlich des Signalpeptides (Glu/Gln 370). Die Marburg II-Mutation hat jedoch keinen Einfluß auf die Prourokinase-aktivierende Aktivität der FSAP.

Die Identifizierung von Personen, die mindestens eine Kopie der FSAP-MR I-Variante tragen, ist nach dem Stand der Technik mittels zwei verschiedener methodischer Ansätze möglich. Ein sicherer Nachweis des Gly/Glu Aminosäureaustausches an Position 534 des Proenzyms (Gly/Glu 534) ist bislang nur durch die Sequenzierung des korrespondierenden codierenden Bereiches in der genomischen DNA oder der mRNA möglich. Ein G/A Basenaustausch in der genomischen Sequenz, der an Nukleotidposition 1601 der cDNA nachgewiesen werden kann, bildet die genetische Ursache für die FSAP-MR 1-Mutante (EP 1 182 258 A1). Auch wenn die DNA-Sequenzanalyse zuverlässige Ergebnisse liefert, so besteht seitens des Routinelabors ein Bedarf an Testverfahren, die möglichst preisgünstig, schnell und sicher sind und die zudem auf vorhandenen Diagnostik-Geräten Geräten automatisch abgearbeitet werden können. Vorwiegend bevorzugt sind immunchemische Nachweis- bzw. Testverfahren, da sie die genannten Kriterien erfüllen und in der Labordiagnostik bereits eine breite Anwendung gefunden haben.

Eine andere Methode zur Bestimmung einer FSAP-MR 1-Mutante beruht auf der Bestimmung des Prourokinase-Aktivierungspotenzials der FSAP in einer Probe. Dazu wird ein spezifischer Antikörper, der nicht in der Lage ist, zwischen Wildtyp-FSAP und den bekannten FSAP-Varianten zu unterscheiden, an eine Festphase gekoppelt und mit der Probenflüssigkeit inkubiert. Nach Zugabe von Prourokinase als FSAP-Substrat und einem chromogenen Urokinase-Substrat, wird die Menge an umgesetztem Chromogen als Maß für die Prourokinase-aktivierende Aktivität der FSAP bestimmt. Träger der Marburg 1-Mutation weisen eine um 50-80 % erniedrigte Prourokinase-aktivierende Aktivität auf. Eine verminderte Prourokinase-aktivierende Aktivität kann jedoch auch durch eine geringe FSAP-Konzentration in der Probe bedingt sein. Daher ist es bislang notwendig, nicht nur die Prourokinase-aktivierende Aktivität, sondern zusätzlich auch die FSAP-Antigenkonzentration einer Probe zu bestimmen (EP 1 348 761 A1 bzw. US 2002/0142316 A1). Im Stand der Technik sind monoklonale Antikörper bekannt, die den immunologischen Nachweis von FSAP ermöglichen. In EP 1 182 258 A1 sind zwei monoklonale Antikörper beschrieben, die den Hybridomazellünien DSM ACC2453 bzw. DSM ACC2454 entstammen, welche nach der Immunisierung von Mäusen mit FSAP-Protein gewonnen wurden. Beide Antikörper binden nicht nur das FSAP-Wildtyp-Protein, sondern ebenso die Marburg I- und II-Varianten. Weitere bekannte FSAP-Antikörper binden gleichermaßen an wildtypisches FSAP wie auch an die bekannten mutanten Varianten; so dass beispielsweise in einem Sandwich-ELISA der Gesamtgehalt an FSAP-Antigen einer Probe bestimmt wird (siehe auch DE 100 23 923 A1). Der Stand der Technik lehrt daher, dass erst wenn eine verminderte Prourokinase-aktivierende Aktivität zusammen mit einer FSAP-Antigenkonzentration im Normbereich festgestellt wird, dies ein konkreter Hinweis auf das Vorhandensein einer FSAP MRI-Variante sei.

Der vorliegenden Erfindung lag die Aufgabe zugrunde weitere Verfahren zur zuverlässigen Diagnose einer MR I-Mutation bzw. zur Erkennung von heterozygoten oder homozygoten Trägern einer MR I-Mutation bereitzustellen, die es ermöglichen auf eine FSAP-Antigenbestimmung zu verzichten.

Die Lösung dieser Aufgabe besteht in der Bereitstellung der in den Ansprüchen beschriebenen erfindungsgemäßen Verfahren, die es ermöglichen, heterozygote oder homozygote Träger eines MR I-Polymorphismus zuverlässig von Nichtträgern zu unterscheiden. Der Vorteil des vorliegenden Verfahrens, das sich die differenzielle Modulation der Aktivität der FSAP-MR I-Variante zu Nutze macht, besteht in einer zuverlässigen Diskriminierung zwischen Trägern und Nichtträgern der FSAP-MR I-Mutation und vor allem darin, dass auf eine zusätzliche Bestimmung des Antigengehalts einer Probe verzichtet werden kann.

Es wurde gefunden, dass durch die Verwendung geeigneter Aktivitätsmodulatoren, sogenannter differenzieller Aktivitätsmodulatoren, die Aktivität der FSAP, insbesondere die amidolytische Aktivität der FSAP gegenüber niedermolekularen Peptidsubstraten sowie die Plasminogenaktivator-aktivierende Aktivität der FSAP, in Proben von Nichtträgern eines MR I-Polymorphismus anders moduliert wird als die Aktivität der FSAP in Proben von hetero- oder homozygoten Trägern eines MR I-Polymorphismus, so dass eine Unterscheidung zwischen Nichtträgern und Trägern eines MR I-Polymorphismus anhand des unterschiedlichen Ausmaßes der Modulation der Aktivität der FSAP getroffen werden kann.

Unter einem differenziellen Aktivitätsmodulator ist im Sinne der vorliegenden Erfindung eine Substanz oder eine Mischung von Substanzen zu verstehen, die entweder

i) die Aktivität der FSAP sowohl in Proben von Trägern der MR I-Variante der FSAP als auch in Proben von Nichtträgern der MR I-Variante der FSAP inhibiert oder verstärkt, dies aber in unterschiedlichem Ausmaß, oder

ii) die Aktivität der FSAP in Proben von Trägern der MR I-Variante der FSAP inhibiert und in Proben von Nichtträgern der MR I-Variante verstärkt oder umgekehrt oder

iii) die Aktivität der FSAP in einem der Probentypen verstärkt oder inhibiert, in dem anderen Probentyp aber keine nennenswerte Veränderung der FSAP-Aktivität bewirkt.

Zur Verwendung als differenzieller Aktivitätsmodulator eignet sich also eine Substanz oder eine Mischung von Substanzen, die

a) einen quantitativen Unterschied bei der Inhibition (Reduktion) oder Verstärkung (Erhöhung) der Aktivität der FSAP in Proben von Nichtträgern bzw. Trägern der MR I-Variante der FSAP bewirkt, d. h. die Aktivität der FSAP wird in beiden Probentypen z. B. erhöht oder reduziert, aber in unterschiedlichem Maße; oder

b) einen qualitativen Unterschied bei der Modulation der Aktivität der FSAP in Proben von Nichtträgern bzw. Trägern der MR I-Variante der FSAP bewirkt, d. h. in einem der Probentypen wird die Aktivität verstärkt, also erhöht, während im anderen Probentyp die Aktivität vermindert, also inhibiert wird.

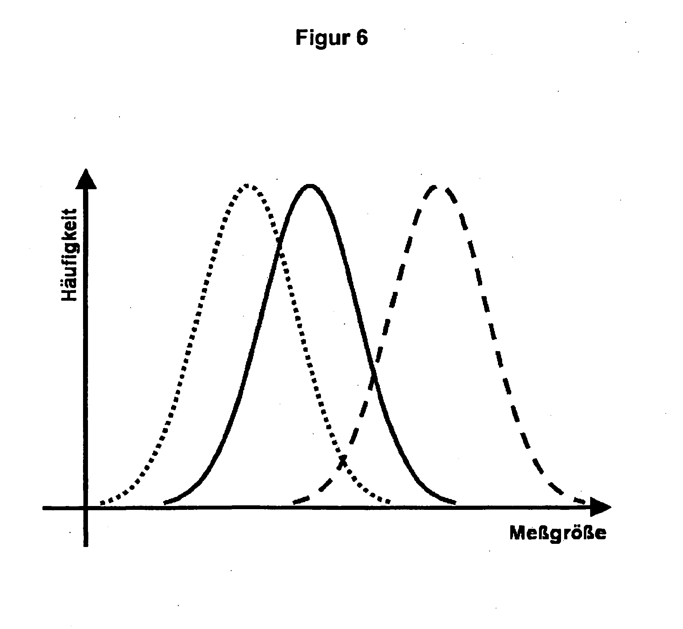

Die unterschiedliche (differenzielle) Modulation der FSAP-Aktivität in Proben von Trägern und Nichtträgern der FSAP-MR I-Variante ermöglicht eine bessere Unterscheidung der beiden Probentypen. Wie in Figur 6 dargestellt, führt dies dazu, dass die Überlappung der Verteilungen der Aktivitäten der FSAP von Proben von Kollektiven von Nichtträgern und Trägern des MR I-Polymorphismus reduziert wird. Damit kann besser zwischen Nichtträgern und den verschiedenen Trägern unterschieden werden, so dass die diagnostische Sensitivität und/oder Spezifität erhöht wird [Vitzthum, F. et al. (2005) Proteomics: from basic research to diagnostic application. A review of requirements & needs. J. Proteome Res. 4(4): 1086-97].

Beispielsweise wird bei der Verwendung von Aprotinin als differenziellem Aktivitätsmodulator ein quantitativer Unterschied beobachtet: die Plasminogenaktivator-aktivierende Aktivität der FSAP in Proben von Nichtträgern wird stärker inhibiert als die Plasminogenaktivator-aktivierende Aktivität der FSAP in Proben von Trägern der FSAP-MR I-Mutation. Homozygote und heterozygote Träger der Wildtypform bzw. der MR I-Variante der FSAP können somit voneinander unterschieden und damit identifiziert werden.

Gegenstand der vorliegenden Erfindung ist also ein in vitro-diagnostisches Verfahren zur Erkennung von Personen mit genetisch bedingter hetero- oder homozygoter Expression der MR I-Variante der FSAP, wobei das Ausmaß der Änderung der Aktivität der FSAP, welche in einer Probe enthalten ist, bestimmt wird. Dazu wird die Aktivität der FSAP, die in einer Probe enthalten ist, in Abwesenheit sowie in Anwesenheit eines differenziellen Aktivitätsmodulators bestimmt, wobei die Bestimmung der FSAP-Aktivität in Abwesenheit und in Anwesenheit entweder

1) parallel, d. h. in zwei gesonderten Reaktionsansätzen, oder

2) sukzessive, d. h. nacheinander in einem einzigen Reaktionsansatz zunächst in Abwesenheit und im Anschluss an die Zugabe des differenziellen Aktivitätsmodulators in Anwesenheit des differenziellen Aktivitätsmodulators durchgeführt werden kann.

Es können zwei bevorzugte Testprinzipien unterschieden werden, die sich zur Bestimmung des Ausmaßes der Änderung der Aktivität der FSAP eignen:

## 1. Bestimmung der Aktivitätsänderung in zwei Reaktionsansätzen

Bei diesem Testprinzip wird das Ausmaß der Änderung der Aktivität der FSAP, die in einer Probe enthalten ist, bestimmt, indem in einem ersten Reaktionsansatz, d. h. in einem ersten Aliquot einer Probe, die Aktivität der FSAP in Anwesenheit eines differenziellen Aktivitätsmodulators der Aktivität der FSAP gemessen wird, und in einem zweiten Reaktionsansatz, d. h. einem zweiten Aliquot derselben Probe, die Aktivität der FSAP in Abwesenheit dieses differenziellen Aktivitätsmodulators gemessen wird. Der Vergleich der Ergebnisse der beiden Reaktionen liefert Aufschluss über das Ausmaß der Änderung der Aktivität der FSAP in Anwesenheit des Aktivitätsmodulators.

Bevorzugterweise wird das Ausmaß der Änderung der Plasminogenaktivator-aktivierenden Aktivität der FSAP einer biologischen Probe, bevorzugt einer Blut- oder Plasmaprobe, bestimmt, indem die Prourokinase-aktivierende Aktivität der FSAP einmal in Abwesenheit und einmal in Anwesenheit eines differenziellen Aktivitätsmodulators anhand der Umsatzkinetik eines Plasminogenaktivators und seines Substrats gemessen wird.

In einer anderen Ausführungsform wird das Ausmaß der Änderung der amidolytischen Aktivität der FSAP einer biologischen Probe, bevorzugt einer Blut- oder Plasmaprobe, bestimmt, indem die amidolytische Aktivität der FSAP einmal in Abwesenheit und einmal in Anwesenheit eines differenziellen Aktivitätsmodulators anhand der Umsatzkinetik eines niedermolekularen FSAP-Substrats gemessen wird.

## 2. Bestimmung der Aktivitätsänderung in einem einzigen Reaktionsansatz

Bei diesem Testprinzip wird das Ausmaß der Änderung der Aktivität der FSAP, die in einer Probe enthalten ist, bestimmt, indem in einem einzigen Reaktionsansatz die Probe mit einem oder mehreren Reagenzien inkubiert wird, welche die FSAP-Aktivitätsbestimmung erlauben, und wobei im Verlaufe der Reaktion ein differenzieller Aktivitätsmodulator hinzugefügt und die resultierende Veränderung der Reaktion verfolgt wird.

Bevorzugterweise wird das Ausmaß der Änderung der Plasminogenaktivator-aktivierenden Aktivität der FSAP einer biologischen Probe, bevorzugt einer Blut- oder Plasmaprobe, bestimmt, indem die Prourokinase-aktivierende Aktivität der FSAP zunächst in Abwesenheit eines differenziellen Aktivitätsmodulators anhand der Umsatzkinetik eines Plasminogenaktivator-Substrats bzw. eines Urokinase-Substrats gemessen wird. Im Verlauf der Reaktion wird dann ein differenzieller Aktivitätsmodulator dem Reaktionsansatz hinzugefügt und die resultierende Veränderung der Reaktion verfolgt.

In einer anderen Ausführungsform wird das Ausmaß der Änderung der amidolytischen Aktivität der FSAP einer biologischen Probe, bevorzugt einer Blut- oder Plasmaprobe, bestimmt, indem die amidolytische Aktivität der FSAP zunächst in Abwesenheit eines differenziellen Aktivitätsmodulators anhand der Umsatzkinetik eines niedermolekularen FSAP-Substrats gemessen wird. Im Verlauf der Reaktion wird dann ein differenzieller Aktivitätsmodulator dem Reaktionsansatz hinzugefügt und die resultierende Veränderung der Reaktion verfolgt.

Unter einer "Probe" ist im Sinne der Erfindung das Material zu verstehen, das die FSAP bzw. die FSAP-MR I-Variante vermutlich enthält. Der Begriff "Probe" umfasst biologische Flüssigkeiten oder Gewebe insbesondere von Menschen und Tieren wie Blut, Plasma, Serum sowie andere Körperflüssigkeiten, Ausscheidungen oder Extrakte, die die FSAP bzw. FSAP-MR I-Mutante vermutlich enthalten. Gegebenenfalls müssen die Proben vorbehandelt werden, um den Analyten für das Nachweisverfahren zugänglich zu machen oder um störende Probenbestandteile zu entfernen. Solche Vorbehandlung von Proben mag die Abtrennung und/oder Lyse von Zellen beinhalten, das Präzipitieren, die Hydrolyse oder die Denaturierung von Probenbestandteilen wie z. B. Proteinen, die Zentrifugation von Proben, das Behandeln der Probe mit organischen Lösungsmitteln wie z. B. Alkohole, insbesondere Methanol; das Behandeln der Probe mit Detergenzien. Häufig wird die Probe in ein anderes, meistens wässriges, Medium überführt, welches mit dem Nachweisverfahren möglichst nicht interferieren soll.

In einer bevorzugten Ausführungsform zur Bestimmung der Aktivität der FSAP wird eine biologische Probe, bevorzugterweise eine Blut- oder Plasmaprobe mit einer Festphase inkubiert, an die zuvor ein Bindungspartner mit Affinität für die FSAP gekoppelt wurde. Zu bevorzugen sind Bindungspartner, die wildtypisches FSAP-Protein mit gleicher Affinität binden wie ein FSAP-Protein mit MR I-Mutation. Bindungspartner, die eine Affinität für die FSAP aufweisen und sich zur Anreicherung bzw. Isolierung von FSAP-Protein aus komplexen Proteinlösungen, wie z. B. Plasmaproben, eignen, sind beispielsweise Substanzen aus der Gruppe Heparin, Heparansulfat, Dextransulfat und Hyaluronsäure. Ebenso geeignet sind monoklonale oder polyklonale Antikörper gegen FSAP oder Antikörper-Fragmente, wie F(ab')- oder F(ab')$_2$-Fragmente. Besonders bevorzugt sind monoklonale Antikörper, die von einer der Hybridomazelllinien DSM ACC2453 und DSM ACC2454 gebildet werden, die bei der DSMZ - Deutsche Sammlung von Mikroorganismen und Zellkulturen GmbH, Mascheroder Weg 1b, 38124 Braunschweig, Deutschland hinterlegt sind.

Der Begriff "Festphase" im Sinne dieser Erfindung beinhaltet einen Gegenstand, der aus porösem und/oder nicht porösem, in der Regel wasserunlöslichem Material besteht und die unterschiedlichsten Formen aufweisen kann, wie z. B. die von Gefäßen, Röhrchen, Mikrotitrationsplatten, Kugeln, Mikropartikeln, Stäbchen, Streifen, Filter- oder Chromatographiepapier. In der Regel ist die Oberfläche der Festphase hydrophil oder kann hydrophil gemacht werden. Die Festphase kann aus den unterschiedlichsten Materialien bestehen wie z. B. aus anorganischen und/oder aus organischen Materialien, aus synthetischen, aus natürlich vorkommenden und/oder aus modifizierten natürlich vorkommenden Materialien. Beispiele für Festphasenmaterialien sind Polymere, wie z. B. Zellulose, Nitrozellulose, Zelluloseacetat, Polyvinylchlorid, Polyacrylamid, vernetzte Dextranmoleküle, Agarose, Polystyrol, Polyethylen, Polypropylen, Polymethacrylat oder Nylon; Keramik, Glas, Metalle, insbesondere Edelmetalle wie Gold und Silber; Magnetit, Mischungen oder Kombinationen derselben etc. Die Festphase kann einen Überzug aus einer oder mehreren Schichten aufweisen z. B. aus Proteinen, Kohlehydraten, lipophilen Substanzen, Biopolymeren, organischen Polymeren oder Mischungen hiervon, um beispielsweise die unspezifische Bindung von Probenbestandteilen an die Festphase zu unterdrücken oder zu verhindern oder um beispielsweise Verbesserungen zu erreichen hinsichtlich der Suspensionsstabilität von partikulären Festphasen, der Lagerstabilität, der formgebenden Stabilität oder der Resistenz gegen UV-Licht, Mikroben oder sonstige zerstörend wirkende Agenzien. Mikropartikel werden häufig als Festphase genutzt. Unter dem Begriff "Mikropartikel" sind im Sinne dieser Erfindung Teilchen zu verstehen, die einen ungefähren Durchmesser von wenigstens 20 nm und nicht mehr als 20 $\mu$m aufweisen, üblicherweise zwischen 40 nm und 10 $\mu$m, bevorzugt zwischen 0,1 und 10 $\mu$m, besonders bevorzugt zwischen 0,1 und 5 $\mu$m, ganz besonders bevorzugt zwischen 0,15 und 2 $\mu$m. Die Mikropartikel können regelmäßig oder unregelmäßig geformt sein. Sie können Kugeln, Spheroide, Kugeln mit mehr oder weniger großen Kavitäten oder Poren darstellen. Die Mikropartikel können aus organischem, aus anorganischem Material oder aus einer Mischung oder Kombination von beiden bestehen. Sie können aus einem porösen oder nicht porösen, einem schwellfähigen oder nicht schwellfähigen Material bestehen. Prinzipiell können die Mikropartikel jegliche Dichte haben. Die Mikropartikel können aus mehreren Schichten bestehen wie beispielsweise die sogenannten "core-and-shell" Partikel mit einem Kern und einer oder mehreren umhüllenden Schichten. Der Begriff Mikropartikel umfasst beispielsweise Farbstoffkristalle, Metallsolen, Silica-Partikel, Glaspartikel, Magnetpartikel, Polymerteilchen, Öltropfen, Lipidpartikel, Dextran und Proteinaggregate, aus Polymermaterial bestehende Partikel, insbesondere aus substituierten Polyethylenen, Latexpartikel z. B. aus Polystyrol, Acrylsäurepolymeren, Methacrylsäurepolymeren, Acrylnitril-Polymeren, AcrylnitrilButadien-Styrol, Polyvinylacetat-Acrylat, Polyvinylpyridin, Vinylchlorid-Acrylat. Von besonderem Interesse sind Partikel mit reaktiven Gruppen an ihrer Oberfläche wie beispielsweise Carboxyl-, Amino- oder Aldehydgruppen, die eine kovalente Bindung z. B. von Bindungspartnern an die Latexpartikel erlauben.

Zur Bestimmung der Plasminogenaktivator-aktivierenden Aktivität der FSAP wird nach Auswaschen der Festphase die an den Bindungspartner gebundene FSAP mit einem inaktiven Einketten-Plasminogenaktivator, wie z. B. Prourokinase oder sct-PA und einem Urokinase-Substrat oder einem sct-PA-Substrat inkubiert. Die FSAP-abhängige Aktivierung von z. B. Prourokinase zu Urokinase wird anhand der Umsetzung bzw. Spaltung des Urokinase-Substrats gemessen. Bevorzugte Substrate sind niedermolekulare Peptidsubstrate, die über eine signalbildende Gruppe verfügen. Die Spaltung des Substrats führt zur Freisetzung der signal bildenden Gruppe. Die physikalischen oder chemischen Eigenschaften der freigesetzten signalbildenden Gruppe unterscheiden sich von den Eigenschaften der an das Peptid gekoppelten Gruppe und können mit Hilfe geeigneter Verfahren quantitativ bestimmt werden. Als signalbildende Gruppe eignen sich z. B. dem Fachmann bekannte Luminophore, Fluorophore oder Chromophore, die mittels optischer Verfahren, wie beispielsweise Lumineszenz-, Fluoreszenz- oder Absorptionsmessungen gemessen werden können. Da die Signalstärke mit der Menge an gespaltenem Substrat korreliert, kann so die Plasminogenaktivator-aktivierende Aktivität der FSAP bestimmt werden. Bevorzugterweise wird ein niedermolekulares Substrat aus der Gruppe S-2444™ (Glu-Gly-Arg-para-Nitroanilin; Chromogenix Instrumentation Laboratory S. p. A., Milano, Italien), Pefachrom® uPA (Ala-Gly-Arg-para-Nitroanilin [Pefa-5221]; Pentapharm Ltd., Basel, Schweiz) und Chromozym™ U (Roche Applied Science, Indianapolis, USA) verwendet.

Zur Bestimmung der amidolytischen Aktivität der FSAP wird nach dem Waschen der Festphase die an den Bindungspartner gebundene FSAP mit einem niedermolekularen Substrat inkubiert und die Umsetzung bzw. Spaltung des Substrats gemessen. Niedermolekulare Substrate im Sinne der vorliegenden Erfindung sind Peptidsubstrate, bestehend aus einer Folge von 2 bis 100, bevorzugt 2 bis 50, besonders bevorzugt 3 bis 10 natürlichen oder unnatürlichen Aminosäuren, die zusätzlich eine signalbildende Gruppe besitzen und die von der FSAP gespalten werden. Die Spaltung eines niedermolekularen Substrats führt zur Freisetzung der signalbildenden Gruppe. Die physikalischen oder chemischen Eigenschaften der freigesetzten signalbildenden Gruppe unterscheiden sich von den Eigenschaften der an das Peptid gekoppelten Gruppe und können mit Hilfe geeigneter Verfahren quantitativ bestimmt werden. Als signalbildende Gruppe eignen sich z. B. dem Fachmann bekannte Luminophore, Fluorophore oder Chromophore, die mittels optischer Verfahren, wie beispielsweise Lumineszenz-, Fluoreszenz- oder Absorptionsmessungen gemessen werden können. Da die Signalstärke mit der Menge an gespaltenem Substrat korreliert, kann so die amidolytische Aktivität der FSAP bestimmt werden. Bevorzugterweise wird ein niedermolekulares, chromogenes Substrat aus der Gruppe Pefa-3297, Pefa-5114, Pefa-5523, Pefa-5773, Pefa-5979, Pefa-3107, Pefa-5329 (alle aus der Pefachrom®-Serie, Pentapharm Ltd., Basel, Schweiz), S-2288™, S-2765™, S-2366™, S-2238™, S-2222™, S-2302™ (alle aus der S-Serie, Chromogenix Instrumentation Laboratory S. p. A., Milano, Italien) verwendet [siehe auch Römisch et al. (1999) Haemostasis 29, 292-299 und Hunfeld et al. (1999) FEBS Letters 456, 290-294].

Die Messung der Substratspaltungsreaktion kann über das komplette Zeitintervall der Reaktion bis zur Gleichgewichtseinstellung oder in mindestens einem bestimmten Zeitintervall oder zu mindestens einem Zeitpunkt erfolgen. Zur Bestimmung der Aktivität können die photometrischen Daten, beispielsweise Spektren oder Absorptionwerte bei bestimmten Wellenlängen, an sich oder in Bezug auf ein Zeitintervall verwendet werden. Wird die Aktivität durch photometrische Daten in Bezug auf ein Zeitintervall bestimmt, d. h. Umsatz- bzw. Reaktionsgeschwindigkeit erfasst, können zur Bestimmung der Umsatz- bzw. Reaktionsgeschwindigkeit verschiedene Verfahren eingesetzt werden. Beispielsweise kann die Umsatzgeschwindigkeit mittels Zeit-Umsatz-Kurven bestimmt werden. Bei Zeit-Umsatz-Kurven wird die Spaltungsprodukt-Konzentration gegenüber der Zeit aufgetragen. Zur Bestimmung der Umsatzgeschwindigkeit wird eine Gerade im Bereich der Reaktion nullter Ordnung der Zeit-Umsatz-Kurve, üblicherweise zu Beginn einer Messung einer Enzymreaktion, angelegt. Die Steigung der Gerade liefert dann die Umsatzgeschwindigkeit, d. h. die Änderung der Konzentration des Substrates oder Produktes in einem bestimmten Zeitintervall [Literatur: Bisswanger, H., Enzymkinetik: Theorie und Methoden, 2. völlig neu bearbeitete Auflage, VCH Verlagsgesellschaft mbH, 1994, Weinheim, NY, Basel, Cambridge, Tokyo; insbesondere S. 66-67].

Messgrößen bzw. Parameter, die sich für die Auswertung der Umsatzkinetik eignen, sind z. B. alle Parameter, die die Reaktionskinetik beschreiben, wie z. B. die Kurvendiskussion per se, insbesondere jedoch einzelne Parameter der Reaktionskinetik, wie die maximale Steigung, d. h. die Reaktionsgeschwindigkeit ($v_{max}$), Sigmoiditätsparameter, Linearitätsparameter, die Fläche unter der Kurve, etc. Parameter, die sich für die Testauswertung eignen, sind z. B. auch absolute Messwerte, wie z. B. Absorptionswerte, die zu einem bestimmten Zeitpunkt gemessen werden oder ein Zeitpunkt, zu dem ein bestimmter Absorptionswert, z. B. ein Maximalwert erreicht ist.

Bei der Bestimmung der FSAP-Aktivitätsänderung in zwei Reaktionsansätzen wird bevorzugterweise die Differenz oder der Quotient eines Testparameters gebildet, der einmal in Abwesenheit und einmal in Gegenwart des Aktivitätsmodulators bestimmt wurde, um das Ausmaß der Änderung der FSAP-Aktivität zu bestimmen. Bei der Verwendung eines Inhibitors, der die FSAP-Aktivität in Proben von Nichtträgern stärker inhibiert als in Proben von MR I-Trägern, ermöglicht beispielsweise die Bildung der Differenz oder des Quotienten aus der Reaktionsgeschwindigkeit ($V_{max}$) der Substratumsatzrate in Abwesenheit des Inhibitors ($V_{max\ 0}$) und der Reaktionsgeschwindigkeit der Substratumsatzrate in Anwesenheit des Inhibitors ($V_{max\ Inhibitor}$) die Differenzierung von FSAP-MR I-Trägern und Nichtträgern. Homozygote bzw. heterozygote Träger der FSAP-MR I-Mutation weisen eine geringere Differenz bzw. einen niedrigeren Quotienten auf als Nichtträger. Andere Algorithmen wie die Summe oder das Produkt sind ebenfalls geeignet, wenn sie eine Differenzierung erlauben.

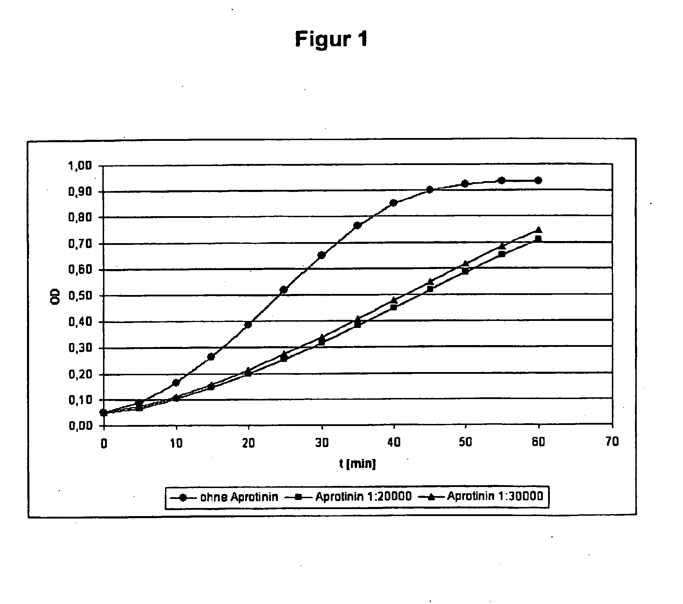

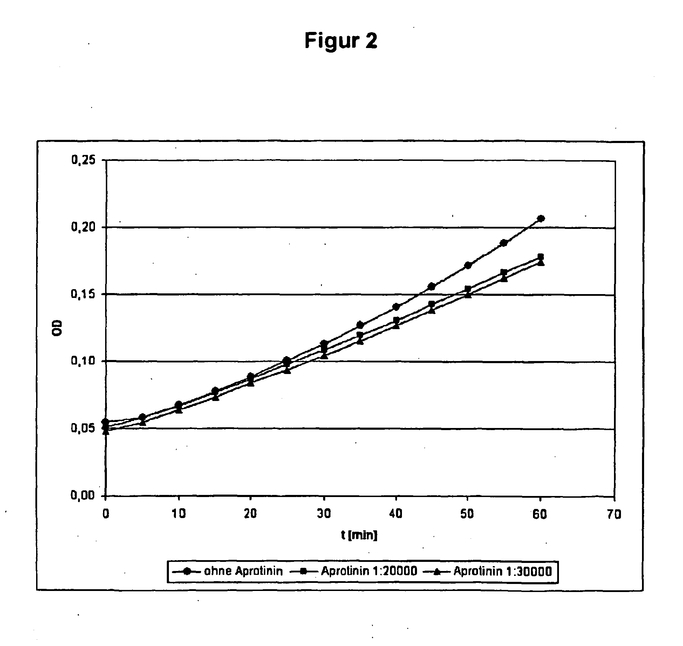

Neben der Reaktionsgeschwindigkeit können auch absolute Testsignale bzw. Messwerte, wie z. B. Absorptionswerte, die zu einem bestimmten Zeitpunkt erreicht werden, verwendet werden (vergleiche z. B. Figuren 1 und 2: ein geeigneter Zeitpunkt in diesem Fall könnte vorzugsweise in einem Zeitintervall zwischen 10 und 70 min, besonders bevorzugt zwischen 30 und 45 min, insbesondere bei 40 min festgelegt werden, da hier eine maximale Differenz zwischen den Ansätzen mit und ohne Aprotinin besteht).

Bei der Bestimmung der FSAP-Aktivitätsänderung in einem einzigen Reaktionsansatz ist es notwendig, die Substratspaltungsreaktion vor und nach der Zugabe des differenziellen Aktivitätsmodulators zu messen. Zumindest ist es notwendig, die Reaktion an mindestens einem Zeitpunkt oder über ein diskretes Zeitintervall vor und an mindestens einem Zeitpunkt oder über ein diskretes Zeitintervall nach Zugabe des differenziellen Aktivitätsmodulators zu messen.

Ein bevorzugter differenzieller Aktivitätsmodulator der Aktivität der FSAP zur Verwendung in dem erfindungsgemäßen Verfahren ist Aprotinin. Aprotinin inhibiert die FSAP-Aktivität in Proben von Nichtträgern stärker als in Proben von MR I-Trägern (siehe Tabelle 3).

Weitere bevorzugte differenzielle Aktivitätsmodulatoren der Aktivität der FSAP zur Verwendung in dem erfindungsgemäßen Verfahren sind monoklonale oder polyklonale anti-FSAP-Antikörper. Besonders bevorzugte monoklonale Antikörper sind solche, die von der Hybridomazelllinie produziert werden, die unter der Hinterlegungsnummer DSM ACC2726( EP 1 630 175 A1) bei der Deutschen Sammlung von Mikroorganismen und Zellkulturen GmbH, Mascheroder Weg 1b, 38124 Braunschweig, Deutschland, hinterlegt ist. Ebenfalls bevorzugt ist ein monoklonaler anti-FSAP-Antikörper, der an ein Epitop der FSAP bindet, welches von einem monoklonalen anti-FSAP-Antikörper, der von der Hybridomazelllinie DSM ACC 2726 gebildet wird, gebunden wird. Diese monoklonalen Antikörper binden an die FSAP und modulieren die FSAP-Aktivität in Proben von Nichtträgern in anderem Ausmaß als in Proben von MR I-Trägern (siehe Tabelle 3).

Um festzustellen, ob sich eine Substanz oder eine Kombination von Substanzen zur Verwendung als differenzieller Aktivitätsmodulator im Sinne der vorliegenden Erfindung eignet, kann wie folgt verfahren werden:

Der Einfluss der zu untersuchenden Substanz oder der zu untersuchenden Kombination von Substanzen auf die Plasminogenaktivator-aktivierende Aktivität der FSAP wird mit Proben bestimmt, die bekanntermaßen die FSAP-MR I-Variante enthalten und mit Proben, die bekanntermaßen die FSAP-MR I-Variante nicht enthalten, aber die FSAP-Wildtypform enthalten. Es kann sich hierbei beispielsweise um Proben von einer oder von mehreren Personen handeln, deren FSAP-Genotyp bekannt ist. Außerdem können Proben verwendet werden, die eine definierte Menge FSAP Wildtyp-Protein bzw. FSAP MR 1-Protein enthalten. Das FSAP-Protein bzw. das FSAP MR I-Protein, das für die Herstellung einer solchen Probe verwendet werden kann, kann z. B. aus menschlichem biologischen Material angereichert oder isoliert sein oder rekombinant oder transgen hergestellt sein. Verfahren zur Anreicherung, Isolierung, Herstellung oder Stabilisierung von FSAP-Protein sind z. B. in den Dokumenten EP 1 226 829 A2, EP 1 074 615 A1 und EP 1 074 616 A1 beschrieben.

Die Plasminogenaktivator-aktivierende Aktivität der FSAP, die in den verschiedenen Proben enthalten ist, wird dann in Abwesenheit sowie in Anwesenheit der zu untersuchenden Substanz bestimmt. Die Substanz oder die Kombination von Substanzen, die auf ihre Eignung als differenzieller Aktivitätsmodulator untersucht werden soll, werden hierbei in mindestens einer Konzentration, vorzugsweise jedoch in verschiedenen Konzentrationen eingesetzt, wie es beispielhaft für Aprotinin in Figur 1 und 2 dargestellt ist.

Das Ausmaß der Änderung der Plasminogenaktivator-aktivierenden Aktivität der FSAP von Proben mit der FSAP-MR I-Variante im Vergleich zu Proben, die die FSAP-MR I-Variante nicht enthalten, aber Aktivität der FSAP-Wildtypform zeigen, erlaubt die Identifizierung von Substanzen bzw. Kombinationen von Substanzen, die als differenzielle Aktivitätsmodulatoren verwendet werden können. Hierbei können Substanzen dadurch als differenzielle Aktivitätsmodulatoren identifiziert werden, dass sie sowohl die Aktivität der FSAP-MR I-Variante als auch die Aktivität der FSAP-Wildtypform inhibieren oder verstärken, dies aber in unterschiedlichem Ausmaß. Eine Substanz ist aber auch dann ein differenzieller Aktivitätsmodulator, wenn sie die Aktivität der FSAP-MR I-Variante inhibiert und die Aktivität der FSAP-Wildtypform verstärkt oder umgekehrt.

Erste Untersuchungen, ob Substanzen als differenzielle Modulatoren geeignet sind, werden mit einer vergleichsweise geringen Anzahl von Proben durchgeführt, beispielsweise mit jeweils mindestens einer Probe der FSAP-MR I-Variante, vorzugsweise jedoch mit jeweils zwei bis zehn Proben. Substanzen, die hierbei eine signifikante, vorzugsweise eine statistisch signifikante Unterscheidung von Proben mit FSAP-Wildtyp und FSAP-MR I liefern, können durch weitere Untersuchungen mit größeren Probenzahlen validiert werden.

Vorzugsweise werden für die weitere Validierung von Substanzen oder Kombinationen von Substanzen die Tests mit Proben von Personen mit bekanntem Genotyp eingesetzt, um realistische Informationen über die Leistungsfähigkeit des Testes zu bekommen, das Vorhandensein bzw. die Abwesenheit einer FSAP-MR I-Variante mit ausreichender diagnostischer Spezifität und Sensitivität zu bestimmen. Die Anzahl der zu untersuchenden Proben einer derartigen Phase I Studie hängt von der zu erwartenden Testgenauigkeit und dem Verhältnis der Anzahl der Proben mit und ohne FSAP-MR I-Variante ab [Obuchowski, N. A. et al. (2004) ROC Curves in Clinical Chemistry: Uses, Misuses and Possible Solutions, Clinical Chemistry, 50:7, 1118-1125]. Bei nahezu perfekter Testgenauigkeit und einem Verhältnis von Proben mit und ohne FSAP-MR I-Variante gleich eins sind jeweils zehn Proben ausreichend, um statistisch signifikante Werte zu erhalten. Die Probenzahl erhöht sich mit zunehmendem und abnehmendem Verhältnis der Proben mit und ohne FSAP-MR I-Variante sowie mit abnehmender Testgenauigkeit. Hier ist es beispielsweise notwendig mehr als jeweils hundert Proben zu untersuchen.

Die Untersuchung von Substanzen oder Kombinationen von Substanzen in Bezug auf ihre Eignung als differenzielle Aktivitätsmodulatoren kann beispielsweise durch die Bestimmung der Prourokinase-aktivierenden Aktivität der FSAP in Anwesenheit und Abwesenheit dieser Substanzen ggf. in verschiedenen Konzentrationen erfolgen. Beispielsweise werden wie in Beispiel 1 Festphasenassoziierte Bindungspartner mit Affinität für die FSAP, insbesondere ein anti-FSAP-Antikörper, der von der Hybridomazelllinie DSM ACC2453 gebildet wird, verwendet und ein Aktivitätsassay wie in Beispiel 1 beschrieben durchgeführt. Die Differenzierung zwischen Trägern und Nichtträgern der FSAP MR 1-Variante wird durch die Bildung eines Quotienten zwischen $v_{max}$ der Reaktionskinetik einer Probe ohne Zusatz ($v_{max\,0}$) und mit Substanzzusatz ($v_{max\,Substanz}$) bestimmt.

Analog kann verfahren werden, wenn Substanzen oder Kombinationen von Substanzen in Bezug auf ihre Eignung als differenzielle Aktivitätsmodulatoren der amidolytischen Aktivität der FSAP untersucht werden sollen. Ein Testverfahren zur Bestimmung der amidolytischen Aktivität der FSAP, wie es in Beispiel 2 beschrieben ist, kann einmal in Abwesenheit und einmal in Gegenwart der zu untersuchenden Substanz durchgeführt werden, und die Testergebnisse können in analoger Weise zur Beurteilung der Eignung einer Substanz verwendet werden, wie soeben beschrieben.

Substanzklassen, die sich insbesondere für eine Untersuchung auf ihre Eignung als differenzielle Aktivitätmodulatoren der Aktivität der FSAP eignen, sind beispielsweise:

    a) Ionen (Anionen wie Chlorid, Carbonat, Sulfat, Phosphat etc. oder Kationen wie Natrium, Lithium, Ammonium, Magnesium, Calcium, Mangan etc.);
    b) Chelatoren wie Ethylendiamintetraacetat (EDTA), Ethylen-Glycol-Bis(β-Aminoethylether)-N,N,N',N'-Tetraacetat, Citrat etc.;

c) Detergenzien wie Natriumdodecylsulfat (SDS), Triton® X 100, Tween® etc.;

d) redoxaktive Substanzen wie Dithioerythrol, Dithiothreitol, β-Mercaptoethanol, Glutathion, Liponsäure, Vitamin C, Vitamin E etc.;

e) Nukleinsäuren, insbesondere Aptamere;

f) Antikörper;

g) Proteine, Peptide und Oligopeptide wie beispielsweise Antithrombin III, C1-Esterase-Inhibitor, tissue factor pathway inhibitor (TFPI), Heparincofaktor II, alpha2-Makroglobulin, alpha2-Antiplasmin, Inter-alpha-Trypsin-Inhibitior, alpha1-Antitrypsin, alpha1-Antichymotrypsin, Plasminogenaktivator Inhibitor Typ 2 (PAI-2), Plasminogenaktivator Inhibitor Typ 3 (PAI-3), Kininogen, hochmolekulares Kininogen (HMWK) etc.;

h) synthetische Serinprotease-Inhibitoren wie FOY-305 [N,N-Dimethylcarbamoylmethyl 4-(4-guanidinobenzoyloxy)-Phenylacetat methansulfonat] und entsprechende Derivate;

i) niedermolekulare Protease-Inhibitoren wie FOIPAN (Camostat Mesilate).

## Figuren

**Figur 1**

Figur 1 zeigt die Inhibition der Prourokinase-aktivierenden Aktivität der FSAP in Anwesenheit von Aprotinin, die für eine Plasmaprobe eines Nichtträgers (Wildtyp) ermittelt wurde.

**Figur 2**

Figur 2 zeigt die Inhibition der Prourokinase-aktivierenden Aktivität der FSAP in Anwesenheit von Aprotinin, die für eine Plasmaprobe eines heterozygoten Trägers des MR 1-Polymorphismus ermittelt wurde.

**Figur 3**

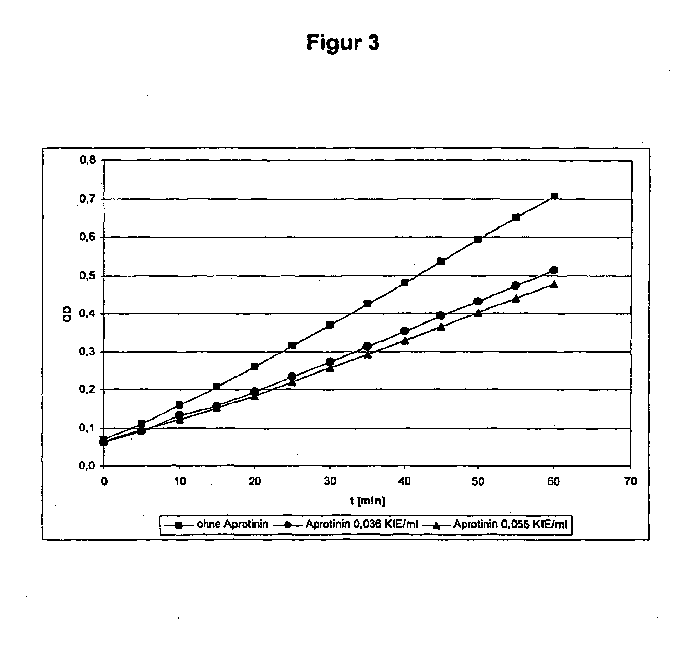

Figur 3 zeigt die Inhibition der amidolytischen Aktivität der FSAP in Anwesenheit von Aprotinin, die für eine Probe eines Plasmapools von Nichtträgern der FSAP-MR I-Mutation (Wildtyp) ermittelt wurden.

**Figur 4**

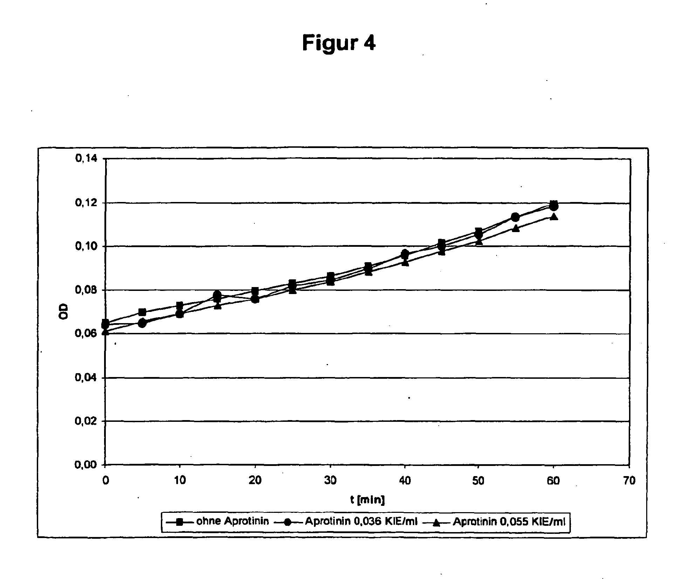

Figur 4 zeigt die Inhibition der amidolytischen Aktivität der FSAP in Anwesenheit von Aprotinin, die für eine Plasmaprobe eines heterozygoten Trägers des MR 1-Polymorphismus ermittelt wurde.

**Figur 5**

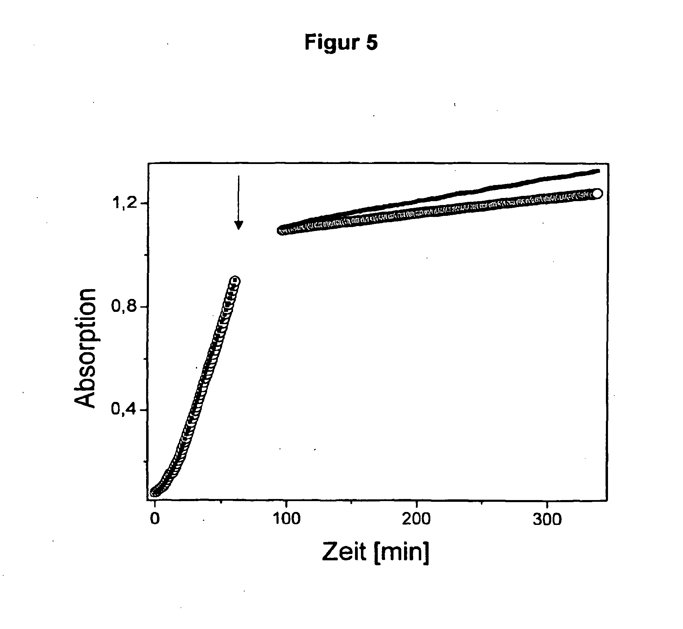

Figur 5 zeigt die Änderung der amidolytischen Aktivität der FSAP für eine Plasmaprobe eines Nichtträgers (Wildtyp; offene Kreise) und eines heterozygoten Trägers des MR I-Polymorphismus (solide Quadrate) in Abwesenheit von Aprotinin und in Anwesenheit von Aprotinin nach dessen Zugabe (Pfeil) bei einer Reaktionszeit von circa einer Stunde, wobei die Aktivität in einem einzigen Reaktionsansatz gemessen wurde. Obwohl die beiden Proben zunächst die gleiche FSAP-Aktivität in Abwesenheit von Aprotinin zeigen und so keine Unterscheidung möglich ist, ändern sich die Aktivitäten in Anwesenheit des Aprotinin, so dass die beiden Proben unterschieden werden können, da die FSAP-Aktivität in der Probe des Nichtträgers stärker inhibiert wird.

**Figur 6**

Figur 6 zeigt beispielhaft eine mögliche Häufigkeitsverteilung einer Messgröße, wie beispielsweise der FSAP-Aktivität in Proben von Kollektiven von homozygoten Trägern der Wildtypform (gestrichelte Linie) und von MR I-Heterozygoten (durchgezogene und gepunktete Linien). Im Fall der gepunkteten Linie wurde ein Modulator verwendet, der die Aktivität der FSAP bei homozygoten Trägern der Wildtypform nicht beeinflusst, die Aktivität der FSAP bei den MR I-Heterozygoten weiter reduziert. Die Überlappung der Verteilungen der Aktivitäten der FSAP von Proben von Kollektiven von Trägern der Wildtypform und von MR I-Heterozygoten ist in Gegenwart des Aktivitätsmodulators geringer als in dessen Abwesenheit. Damit kann in Gegenwart des Aktivitätsmodulators besser zwischen den verschiedenen Trägern bzw. Formen unterschieden werden als in dessen Abwesenheit, so dass die diagnostische Sensitivität und/oder Spezifität erhöht wird.

Die im Folgenden beschriebenen Beispiele dienen der exemplarischen Beleuchtung einzelner Aspekte dieser Erfindung und sind nicht als Einschränkung zu verstehen:

## Beispiel 1

**Bestimmung der Prourokinase-aktivierenden Aktivität der FSAP in Anwesenheit und in Abwesenheit des differenziellen Aktivitätsmodulators Aprotinin**

Als Festphasen-assoziierter Bindungspartner mit Affinität für die FSAP wurde ein anti-FSAP-Antikörper, der von der Hybridomazelllinie DSM ACC2453 gebildet wird, verwendet. Zur Durchführung des heterogenen Nachweisverfahrens wurden Mikrotiterplatten (MTPs) aus Polystyrol als Festphase verwendet. An die Polystyrol-Festphase wurde über Nacht bei Raumtemperatur anti-FSAP-Antikörper in 50 mM NaHCO$_3$, pH 8,2 bei einem Beschichtungsvolumen von 120 µl pro Näpfchen der MTP und einer Beschichtungskonzentration von 20 µg Antikörper pro ml assoziiert. Ungebundene Antikörper wurden durch dreimaliges Waschen mit 50 mM Natrium-Phosphat-gepufferter, isotonischer NaCl-Lösung, 0,02 % Tween® 20, pH 6,5 entfernt.

In die Vertiefungen wurden jeweils 100 $\mu$l einer zu bestimmenden Plasmaprobe in einer Verdünnung von 1: 80 in Probenpuffer (20 mM Natriumcitrat, pH 6,0 mit 150 mM NaCl, 100 mM L-Argininmonohydrochlorid, 1 % Rinderserumalbumin, 0,1 % Tween® 80, 100 I.E. Heparin/ml) pipettiert. Nach Inkubation bei +37 ˚C für eine Stunde wurden ungebundene Bestandteile durch dreimaliges Waschen mit 50 mM Natrium-Phosphat-gepufferter, isotonischer NaCl-Lösung, 0,02 % Tween® 20, pH 6,5 entfernt.

Zur Bestimmung der Prourokinase-aktivierenden Aktivität der FSAP wurden nach Entfernen der Probe und Auswaschen der Festphase

a) 30 $\mu$l Testpuffer I (50 mM Tris/HCl, pH 7,2 mit 150 mM NaCl, 0,2 % Tween® 80, 15 mM CaCl$_2$ und 50 I.E./ml Heparin) oder

b) 30 $\mu$l Testpuffer I, der zusätzlich Aprotinin in einer solchen Konzentration enthielt, dass eine finale Konzentration von 0,055 KIE/ml Aprotinin im Reaktionsansatz erzielt wurde (1 U = 1 Kallikrein Inhibierende Einheit [KIE]; Aprotinin aus Rinderlunge, Sigma-Aldrich Laborchemikalien GmbH, Taufkirchen, Deutschland)

und jeweils 50 $\mu$l rekombinante Prourokinase (Landing Biotech Inc., Brighton, MA, USA; 5 $\mu$g/ml in Testpuffer I) und jeweils 50 $\mu$l des chromogenen Substrates S-2444™ (0,6 mM) in Testpuffer II (100 mM Tris/HCl, 150 mM NaCl, 15 mM Na-Azide, 0,1 % Tween® 80, pH 8,2) in ein MTP-Näpfchen gegeben und bei +37 ˚C inkubiert. Die Absorptionsänderung (OD) der Reaktionsansätze wurde bei einer Wellenlänge von 405 nm verfolgt. Die Ergebnisse sind in Tabelle 1 zusammengefasst. Eine Differenzierung zwischen Trägern und Nichtträgern der FSAP MR I-Variante ist möglich durch die Bildung eines Quotienten zwischen $V_{max}$ der Reaktionskinetik einer Probe ohne Aprotininzusatz ($v_{max0}$) und mit Aprotininzusatz ($V_{max\ Aprotinin}$). Hierfür gilt:

$$\text{FSAP-Wildtyp-}v_{max0} / \text{FSAP-Wildtyp-}v_{max\ Aprotinin} > \text{FSAP-MR I-}v_{max0} / \text{FSAP-MR I-}v_{max\ Aprotinin}.$$

Ausserdem zeigen FSAP-MR I-Proben (MR I) eine deutlich geringere Differenz zwischen $v_{max0}$ und $v_{max\ Aprotinin}$ als FSAP-Wildtyp-Proben (WT) (siehe Tabelle 1). In der Spalte MW ist der Mittelwert der drei Einzelbestimmungen dargestellt.

Die maximale Steigung der Zeit-Umsatz-Kurven wurde mittels Linearregression in einem ausreichend linearen Bereich der Zeit-Umsatz-Kurve bestimmt und lieferte die maximale Reaktionsgeschwindigkeit $V_{max}$ einer Reaktion in mOD/min.

**Tabelle 1**

| Proben-nr.: | FSAP- | Ohne Aprotinin $Vmax_0$ | 0,055 KIE/ml Aprotinin $Vmax_{Aprotinin}$ | $Vmax_0/Vmax_{Aprotinin}$ | MW $Vmax_D/Vmax_{Aprotinin}$ | $Vmax_0 - Vmax_{Aprotinin}$ | MW $Vmax_D \cdot Vmax_{Aprotinin}$ |
|---|---|---|---|---|---|---|---|
| 14942 | MR1 | 3,04 | 2,00 | 1,52 | | 1,04 | |
| 229 | MR1 | 2,48 | 1,84 | 1,35 | 1,43 | 0,64 | 0,90 |
| 808 | MR1 | 3,45 | 2,45 | 1,41 | | 1,01 | |
| 2173 | WT | 23,07 | 11,84 | 1,95 | | 11,23 | |
| 2175 | WT | 26,23 | 13,82 | 1,90 | 2,01 | 12,42 | 11,37 |
| 2185 | WT | 21,47 | 12,09 | 1,78 | | 9,38 | |
| 2196 | WT | 21,25 | 8,81 | 2,41 | | 12,44 | |

Die Figuren 1 und 2 zeigen die unterschiedlichen Reaktionskinetiken der Umsetzung von S-2444™, also die Prourokinase-aktivierende Aktivität der FSAP. Figur 1 zeigt die Reaktionskinetiken, die für eine Plasmaprobe eines Nichtträgers ohne bzw. mit Zusatz von Aprotinin (0,036 und 0,055 KIE/ml, finale Aprotininkonzentration) ermittelt wurden. Hierbei entsprechen 0,036 KIE/ml Aprotinin einer Aprotininverdünnung von 1:30000 und 0,055 KIE/ml einer Aprotininverdünnung von 1:20000. Figur 2 zeigt die Reaktionskinetiken, die für eine Plasmaprobe eines heterozygoten Trägers der FSAP-MR I-Variante ohne bzw. mit Zusatz von Aprotinin (0,036 und 0,055 KIE/ml finale Aprotininkonzentration) ermittelt wurden. Es ist deutlich zu erkennen, dass die Prourokinase-aktivierende Aktivität der FSAP in der Probe des Nichtträgers in Gegenwart von Aprotinin wesentlich stärker inhibiert wird als die Prourokinase-aktivierende Aktivität der FSAP in der Probe des heterozygoten Trägers.

## **Beispiel 2**

**Bestimmung der amidolytischen Aktivität der FSAP gegenüber dem niedermolekularen, chromogenen Peptidsubstrat S-2288™ in Anwesenheit und in Abwesenheit des differenziellen Aktivitätsmodulators Aprotinin**

Es wurden die gleichen Mikrotiterplatten verwendet wie in Beispiel 1 beschrieben. In die Vertiefungen wurden jeweils 100 µl einer zu bestimmenden Plasmaprobe in einer Verdünnung von 1:50 in Probenpuffer (siehe Beispiel 1) pipettiert. Nach Inkubation bei +37˚C für eine Stunde wurden ungebundene Bestandteile durch dreimaliges Waschen mit 50 mM Natrium-Phosphat-gepufferter isotonischer NaCl-Lösung, 0,02 % Tween® 20, pH 6,5 entfernt.

Zur Bestimmung der amidolytischen Aktivität der FSAP wurden nach Entfernen der Probe und Auswaschen der Festphase

a) 30 µl Testpuffer I (siehe Beispiel 1) oder
b) 30 µl Testpuffer I, der zusätzlich Aprotinin in einer solchen Konzentration enthielt, dass eine finale Konzentration von 0,055 KIE/ml Aprotinin im Reaktionsansatz erzielt wurde (siehe Beispiel 1),
und jeweils 80 µl des chromogenen Substrates S-2288™ (1,5 mmol/L; Chromogenix Instrumentation Laboratory S. p. A., Milano, Italien) in Testpuffer II (siehe Beispiel 1) in die Testvertiefung gegeben und für eine Stunde bei +37 ˚C inkubiert. Um Verdunstungseffekte während der relativ langen Inkubationsdauer in der MTP zu reduzieren, wurde mit Mineralöl überschichtet.

Die Absorptionsänderung (OD) der Reaktionsansätze wurde bei einer Wellenlänge von 405 nm verfolgt, und es wurde die maximale Reaktionsgeschwindigkeit $V_{max}$ bestimmt (siehe Beispiel 1). Die Ergebnisse sind in Tabelle 2 zusammengefasst.
Eine Differenzierung zwischen Trägern und Nichtträgern der FSAP MR I-Variante ist möglich durch die Bildung eines Ratios (Quotienten) zwischen $V_{max}$ der Reaktionskinetik einer Probe ohne Aprotininzusatz ($V_{max0}$) und mit Aprotinin-zusatz ($V_{max\ Aprotinin}$). Hierfür gilt:

$$\text{FSAP-Wildtyp-}V_{max0} / \text{FSAP-Wildtyp-}V_{max\ Aprotinin} > \text{FSAP-MR I-}V_{max0} / \text{FSAP-MR I-}V_{max\ Aprotinin}.$$

Ausserdem zeigen FSAP-MR I-Proben (MR I) eine deutlich geringere Differenz zwischen $V_{max0}$ und $V_{max\ Aprotinin}$ als FSAP-Wildtyp-Proben (WT) (siehe Tabelle 2). In der Spalte MW ist der Mittelwert der drei Einzelbestimmungen dargestellt.

**Tabelle 2**

| Proben-nr.: | FSAP- | Ohne Aprotinin $Vmax_0$ | 0,055 KIE/ml Aprotinin $Vmax_{Aprotinin}$ | $Vmax_0 / Vmax_{Aprotinin}$ | MW $Vmax_D / Vmax_{Aprotinin}$ | $Vmax_0 -Vmax_{Aprotinin}$ | MW $Vmax_D -Vmax_{Aprotinin}$ |
|---|---|---|---|---|---|---|---|
| 14942 | MR I | 1,24 | 1,22 | 1,02 | | 0,02 | |
| 7020538 | MR I | 0,97 | 0,94 | 1,03 | 1,04 | 0,03 | 0,07 |
| 7020551 | MR I | 2,02 | 1,87 | 1,08 | | 0,15 | |
| WT P2 | WT | 11,37 | 7,52 | 1,51 | | 3,86 | |
| 305 | WT | 9,99 | 6,90 | 1,45 | 1,48 | 3,09 | 3,81 |
| 2176 | WT | 14,06 | 9,57 | 1,47 | | 4,49 | |

Die Figuren 3 und 4 zeigen die unterschiedlichen Reaktionskinetiken der Umsetzung von S-2288™, also die amidolytische Aktivität der FSAP. Figur 3 zeigt die Reaktionskinetiken, die für eine Plasmaprobe eines Nichtträgers der MR I-Mutation ohne bzw. mit Zusatz von Aprotinin (0,036 und 0,055 KIE/ml finale Aprotininkonzentration) ermittelt wurden. Figur 4 zeigt die Reaktionskinetiken, die für eine Plasmaprobe eines heterozygoten Trägers der FSAP-MR I-Variante ohne bzw. mit Zusatz von Aprotinin (0,036 und 0,055 KIE/ml finale Aprotininkonzentration) ermittelt wurden. Es ist deutlich zu erkennen, dass die amidolytische Aktivität der FSAP in der Probe des Nichtträgers in Gegenwart von Aprotinin wesentlich stärker inhibiert wird als die amidolytische Aktivität der FSAP in der Probe des heterozygoten Trägers.

**Beispiel 3**

**Bestimmung der Änderung der amidolytischen Aktivität der FSAP gegenüber dem niedermolekularen, chromogenen Peptidsubstrat S-2288™ durch Zugabe eines differenziellen Aktivitätsmodulators im Verlauf der Reaktion**

Es wurden die gleichen Mikrotiterplatten verwendet wie in Beispiel 1 beschrieben. In die Vertiefungen wurden jeweils 100 $\mu$l einer zu bestimmenden Plasmaprobe in einer Verdünnung von 1:15 in Probenpuffer (siehe Beispiel 1) pipettiert. Nach Inkubation bei +37 ˚C für eine Stunde wurden ungebundene Bestandteile durch dreimaliges Waschen wie in Beispiel 1 entfernt.

Zur Bestimmung der amidolytischen Aktivität der FSAP wurden zum Zeitpunkt $t_0$ jeweils 80 $\mu$l des chromogenen Substrates S-2288™ (1,5 mmol/L; Chromogenix Instrumentation Laboratory S. p. A., Milano, Italien) in einem 50:50-Gemisch aus Testpuffer I und Testpuffer II (Testpuffer I und 11 siehe Beispiel 1) in die MTP-Näpfchen gegeben und bei +37 ˚C im Photometer inkubiert. Nach etwa 60 Minuten wurde Aprotinin in einer finalen Konzentration von 2,95 KIE/ml oder der jeweilige monoklonale Antikörper (MAK), in einer Endkonzentration von circa 60 $\mu$g/ml pro Reaktionsansatz zugegeben. Die Absorptionsänderung der Reaktionsansätze wurde bei einer Wellenlänge von 405 nm verfolgt, und es wurde die maximale Reaktionsgeschwindigkeit $_{Vmax}$ bestimmt (siehe Beispiel 1). Um Verdunstungseffekte während der relativ langen Inkubationsdauer zu reduzieren, wurde mit Mineralöl überschichtet.

Eine Differenzierung zwischen Trägern und Nichtträgern der FSAP MR I-Variante ist möglich durch die Bildung eines Verhältnisses, beispielsweise des Quotienten aus der maximalen Reaktionsgeschwindigkeit vor der Zugabe des differenziellen Aktivitätsmodulators ($v_{max}$) und der maximalen Reaktionsgeschwindigkeit nach Zugabe des differenziellen Aktivitätsmodulators ($V_{max\ Aprotinin}$).

Entsprechend Figur 5 gilt für den Aktivitätsmodulator Aprotinin:

$$\text{FSAP-Wildtyp-}v_{max} \text{ / FSAP-Wildtyp-}v_{max\ Aprotinin} > \text{FSAP-MR I-}v_{max} \text{ / FSAP-MR I-}v_{max\ Aprotinin}.$$

In Tabelle 3 ist neben Aprotinin auch der Einfluss von monoklonalen Antikörpern (MAKS) auf den Quotienten $V_{max}/V_{max}$ Aktivitätsmodulator dargestellt. Bei der Probe des Nichtträgers der FSAP MR I-Variante (Wildtypprobe; WT) wurden 3 unabhängige Messungen durchgeführt. Bei der Probe des heterozygoten Trägers der FSAP MR I-Variante (MR I) wurden 6 unabhängige Messungen durchgeführt. Es wurden die Mittelwerte (MW) von $V_{max}/V_{maxAktivitätsmodulator}$ ($V_{max}/V_{maxA}$) und die Standardabweichungen bezogen auf die Grundgesamtheit (Staw) berechnet. Die Negativkontrolle, d.h. die Zugabe eines entsprechenden Volumens von Testpuffer I und Testpuffer II im Verhältnis 50:50 ohne Aktivitätsmodulator, lieferte einen MW $V_{max}/V_{maxA}$ von 0,81 +/- 0,12.

Um einen Hinweis auf die Signifikanz des Einflusses eines Aktivitätsmodulators zu erhalten, wurde ein t-Test durchgeführt. Der t-Test basierte auf der Annahme von zwei Stichproben mit gleicher und ungleicher Varianz. Es wurde ein einseitiger Test (eine Endfläche) durchgeführt. Dieser t-Test zeigt, dass Aprotinin, der monoklonale anti-FSAP-Antikörper MAK 2004-151/013(2) (DSM ACC2726) und der monoklonale anti-FSAP-Antikörper MAK 2004-98/016(3), letzterer dient hier lediglich illustrativen Zwecken und ist nicht Teil der Erfindung, mit entsprechender Signifikanz die Aktivität der WT-Probe relativ zur MRI-Probe stärker reduziert, so dass der WT/MRI-Quotient > 1 ist. Der monoklonale anti-FSAP-Antikörper MAK 1102/1189-2 (DSM ACC2454) hingegen reduziert die Aktivität der MR I-Probe relativ zur WT-Probe stärker, so dass der WT/MR I-Quotient < 1 ist. Dies ist auch bei dem monoklonalen anti-FSAP-Antikörper MAK 2004-35/05(1) (DSM ACC2674) der Fall. Da die P-Werte hier zwar noch keine Signifikanz anzeigen aber vergleichsweise niedrig sind, kann davon ausgegangen werden, dass bei einer ausreichenden Anzahl von Messungen, d. h. bei einer größeren Fallzahl, die Nullhypothese ausgeschlossen werden kann. MAK 1102/1189-2 (DSM ACC 2454) und MAK 2004-35/05 (1) (DSM ACC 2674), die illustrativen Zwecken dienen und nicht Teil der Erfindung sind, können sich ebenfalls als Aktivitätsmodulatoren eignen.

Im Gegensatz dazu sind die monoklonalen anti-FSAP-Antikörper MAK 1102/570-09 (DSM ACC2533, siehe EP 1 334 983 A2), MAK 2004-9/026(2) (DSM ACC2676, siehe EP 1 630 175 A1) und MAK 2004-34/08(2) (DSM ACC2725, siehe EP 1 630 175 A1) ihren WT/MR I-Quotienten und ihren t-Test-Werten zufolge nicht als Aktivitätsmodulatoren geeignet.

**Tabelle 3**

| Aktivitätsmodulator | WT | | MRI | | WT/MRI | P-Wert für gleiche Varianz | P-Wert für ungleiche Varianz |
|---|---|---|---|---|---|---|---|
| | MW $V_{max}/V_{max\,A}$ | Staw | MW $V_{max}/V_{max\,A}$ | Staw | | | |
| Aprotinin | 16,72 | 1,38 | 12,36 | 2,31 | 1,35 | 0,0331 | 0,0224 |
| MAK 1102/570-09 (DSM ACC2533) | 1,32 | 0,20 | 1,36 | 0,15 | 0,97 | 0,7947 | 0,8293 |
| MAK 1102/1189-2* (DSM ACC2454) | 0,72 | 0,03 | 0,78 | 0,04 | 0,92 | 0,0757 | 0,0625 |
| MAK 2004-9/026(2) (DSM ACC2676) | 0,71 | 0,05 | 0,76 | 0,03 | 0,93 | 0,1109 | 0,2555 |
| MAK 2004-35/05(1)* (DSM ACC2674) | 0,71 | 0,07 | 0,87 | 0,19 | 0,82 | 0,2596 | 0,1531 |
| MAK 2004-34/08(2) (DSM ACC2725) | 0,74 | 0,05 | 0,77 | 0,06 | 0,95 | 0,4143 | 0,4229 |
| MAK 2004-151/013 (2) (DSM ACC2726) | 0,95 | 0,04 | 0,80 | 0,05 | 1,19 | 0,0057 | 0,0119 |
| MAK 2004-98/016(3)* | 0,94 | 0,05 | 0,77 | 0,06 | 1,23 | 0,0063 | 0,0109 |
| *Antikörper dienen lediglich illustrativen Zwecken und sind nicht Teil der Erfindung. | | | | | | | |

**Patentansprüche**

1. Diagnostisches Verfahren zur Erkennung von Personen mit genetisch bedingter hetero- oder homozygoter Expression der MR I-Variante der Faktor VII-aktivierenden Protease (FSAP), bei dem die Aktivität der FSAP bestimmt wird, **dadurch gekennzeichnet, dass** die FSAP-Aktivität, die in einer Probe enthalten ist, in Abwesenheit sowie in Anwesenheit eines differenziellen Aktivitätsmodulators bestimmt wird, wobei der differenzielle Aktivitätsmodulator die Aktivität der FSAP- in Proben von Personen mit genetisch bedingter hetero- oder homozygoter Expression der MR I-Variante der FSAP in anderem Ausmaß ändert als in Proben von Personen, die die MRI-Variante der FSAP nicht exprimieren.

2. Verfahren nach Anspruch 1, bei dem die Aktivität der FSAP, die bestimmt wird, die Plasminogenaktivator-aktivierende Aktivität der FSAP- ist.

3. Verfahren nach Anspruch 1, bei dem die Aktivität der FSAP, die bestimmt wird, die Aktivität der FSAP ist.

4. Verfahren nach einem der Ansprüche 1 bis 3, wobei der differenzielle Aktivitätsmodulator die FSAP-Aktivität in Proben von Personen, die die MRI-Variante der FSAP nicht exprimieren, in stärkerem Ausmaß inhibiert oder aktiviert als in Proben von Personen mit genetisch bedingter hetero- oder homozygoter Expression der MR I-Variante der FSAP.

5. Verfahren nach einem der vorhergehenden Ansprüche, wobei als differenzieller Aktivitätsmodulator Aprotinin verwendet wird.

6. Verfahren nach einem der vorhergehenden Ansprüche, wobei als differenzieller Aktivitätsmodulator ein monoklonaler oder polyklonaler anti-FSAP-Antikörper verwendet wird.

**7.** Verfahren nach Anspruch 6, wobei ein monoklonaler anti-FSAP-Antikörper verwendet wird, der von der Hybridomazelllinie DSM ACC2726 gebildet wird.

**8.** Verfahren nach Anspruch 6, wobei ein monoklonaler anti-FSAP-Antikörper verwendet wird, der an ein Epitop der FSAP bindet, das von einem monoklonalen anti-FSAP-Antikörper gebunden wird, der von der Hybridomazelllinie DSM ACC2726 gebildet wird.

**9.** Verfahren nach einem der Ansprüche 1 bis 8, bei dem das Ausmaß der Änderung der FSAP-Aktivität bestimmt wird durch:

> a) Bestimmung der FSAP-Aktivität in einem ersten Aliquot einer Probe in Anwesenheit des differenziellen Aktivitätsmodulators;
> b) Bestimmung der FSAP-Aktivität in einem zweiten Aliquot derselben Probe in Abwesenheit des differenziellen Aktivitätsmodulators; und
> c) Vergleich der beiden in a) und b) bestimmten Aktivitäten.

**10.** Verfahren nach einem der Ansprüche 1 bis 8, bei dem das Ausmaß der Änderung der FSAP-Aktivität bestimmt wird durch:

> a) Bestimmung der FSAP Aktivität in einem Aliquot einer Probe in Abwesenheit des differenziellen Aktivitätsmodulators; dann
> b) Zugabe des differenziellen Aktivitätsmodulators zum Reaktionsansatz; dann
> c) Bestimmung der FSAP-Aktivität in Anwesenheit des differenziellen Aktivitätsmodulators; und
> d) Vergleich der beiden in a) und c) bestimmten Aktivitäten.

**Claims**

**1.** A diagnostic method for identifying persons with genetically related hetero- or homozygous expression of the MR I variant of factor VII-activating protease (FSAP), in which the activity of FSAP is determined, which comprises the FSAP activity present in a sample being determined in the absence and in the presence of a differential activity modulator, where the differential activity modulator changes the FSAP activity in samples from persons with genetically related hetero- or homozygous expression of the MR I variant of FSAP to a different extent than in samples from persons who do not express the MR I variant of FSAP.

**2.** The method as claimed in claim 1, in which the FSAP activity which is determined is the plasminogen activator-activating activity of FSAP.

**3.** The method as claimed in claim 1, in which the FSAP activity which is determined is the amidolytic activity of FSAP.

**4.** The method as claimed in any of claims 1 to 3, where the differential activity modulator inhibits or activates the FSAP activity in samples from persons who do not express the MR I variant of FSAP to a greater extent than in samples from persons with genetically related hetero- or homozygous expression of the MR I variant of FSAP.

**5.** The method as claimed in any of the preceding claims, where aprotinin is used as differential activity modulator.

**6.** The method as claimed in any of the preceding claims, where a monoclonal or polyclonal anti-FSAP antibody is used as differential activity modulator.

**7.** The method as claimed in claim 6, where a monoclonal anti-FSAP antibody which is produced by the hybridoma cell line DSM ACC2726 is used.

**8.** The method as claimed in claim 6, where a monoclonal anti-FSAP antibody which binds to an FSAP epitope which is bound by a monoclonal anti-FSAP antibody which is produced by the hybridoma cell line DSM ACC2726 is used.

**9.** The method as claimed in any of claims 1 to 8, in which the extent of the change in the FSAP activity is determined by:

> a) determining the FSAP activity in a first aliquot of a sample in the presence of the differential activity modulator;

b) determining the FSAP activity in a second aliquot of the same sample in the absence of the differential activity modulator; and

c) comparing the two activities determined in a) and b).

**10.** The method as claimed in any of claims 1 to 8, in which the extent of the change in the FSAP activity is determined by:

a) determining the FSAP activity in an aliquot of a sample in the absence of the differential activity modulator; then

b) adding the differential activity modulator to the reaction mixture; then

c) determining the FSAP activity in the presence of the differential activity modulator; and

d) comparing the two activities determined in a) and c).


**Revendications**

**1.** Procédé de diagnostic pour la détection de personnes ayant une expression génétique hétérozygote ou homozygote de la variante MRI de la protéase activant le facteur VII (FSAP), dans lequel on détermine l'activité du FSAP, **caractérisé en ce que** l'on détermine l'activité du FSAP qui est contenu dans un échantillon, en l'absence ainsi qu'en la présence d'un modulateur différentiel d'activité, le modulateur différentiel d'activité modifiant l'activité du FSAP dans des échantillons de personnes ayant une expression génétique hétérozygote ou homozygote de la variante MRI du FSAP dans une mesure autre que dans des échantillons de personnes qui n'expriment pas la variante MRI du FSAP.

**2.** Procédé suivant la revendication 1, dans lequel l'activité du FSAP, qui est déterminée, est l'activité du FSAP activant l'activateur plasminogène.

**3.** Procédé suivant la revendication 1, dans lequel l'activité du FSAP, qui est déterminée, est l'activité amidolytique du FSAP.

**4.** Procédé suivant l'une des revendications 1 à 3, dans lequel le modulateur différentiel d'activité inhibe ou active l'activité FSAP dans des échantillons de personnes qui n'expriment pas la variante MRI du FSAP dans une mesure plus forme que dans des échantillons de personnes ayant une expression génétique hétérozygote ou homozygote de la variante MRI du FSAP.

**5.** Procédé suivant l'une des revendications précédentes, dans lequel on utilise de l'aprotinine comme modulateur différentiel d'activité.

**6.** Procédé suivant l'une des revendications précédentes, dans lequel on utilise un anticorps anti-FSAP monoclonal ou polyclonal comme modulateur différentiel d'activité.

**7.** Procédé suivant la revendication 6, dans lequel on utilise un anticorps anti-FSAP monoclonal qui est formé par la lignée hybridomazelle DSM ACC2726.

**8.** Procédé suivant la revendication 6, dans lequel on utilise un anticorps anti-FSAP monoclonal qui fixe un épitope du FSAP qui est fixé par un anticorps anti-FSAP monoclonal, lequel est formé par la lignée hybridomazelle DSM ACC2726.

**9.** Procédé suivant l'une des revendications 1 à 8, dans lequel on détermine la mesure de la modification de l'activité FSAP :

a) en déterminant l'activité FSAP dans un premier aliquote d'un échantillon en présence du modulateur différentiel d'activité ;

b) en déterminant l'activité FSAP dans un deuxième aliquote du même échantillon en l'absence du modulateur différentiel d'activé ; et

c) en comparant les deux activités déterminées dans a) et b).

**10.** Procédé suivant l'une des revendications 1 à 8, dans lequel on détermine la mesure de la variation de l'activité FSAP :

a) en déterminant l'activité FSAP dans un aliquote d'un échantillon en l'absence du modulateur différentiel d'activité ; puis

b) en ajoutant le modulateur différentiel d'activité à la masse réactionnelle ; puis

c) en déterminant l'activité FSAP en présence du modulateur différentiel d'activité ; et

d) en comparant les deux activités déterminées dans a) et c).

# Figur 1

# Figur 2

# Figur 3

# Figur 4

# Figur 5

**Figur 6**

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 952215 A2
- EP 1182258 A1
- EP 1348761 A1
- US 20020142316 A1
- DE 10023923 A1
- EP 1630175 A1
- EP 1226829 A2
- EP 1074615 A1
- EP 1074616 A1
- EP 1334983 A2

**In der Beschreibung aufgeführte Nicht-Patentliteratur**

- **WILLEIT et al.** Marburg I polymorphism of factor VI-lactivating protease: a prominent risk predictor of carotid stenosis. *Circulation,* 2003, vol. 107, 667-670
- **RÖMISCH et al.** The FVII activating protease cleaves single-chain plasminogen activators. *Haemostasis,* 1999, vol. 29, 292-299
- **HUNFELD et al.** Detection of a novel plasma serine protease during purification of vitamin K-dependent coagulation factors. *FEBS Letters,* 1999, vol. 456, 290-294
- **VITZTHUM, F. et al.** Proteomics: from basic research to diagnostic application. A review of requirements & needs. *J. Proteome Res.,* 2005, vol. 4 (4), 1086-97
- **Römisch et al.** *Haemostasis,* 1999, vol. 29, 292-299
- **Hunfeld et al.** *FEBS Letters,* 1999, vol. 456, 290-294
- **BISSWANGER, H.** Enzymkinetik: Theorie und Methoden. VCH Verlagsgesellschaft mbH, 1994, 66-67
- **OBUCHOWSKI, N. A. et al.** ROC Curves in Clinical Chemistry: Uses, Misuses and Possible Solutions. *Clinical Chemistry,* 2004, vol. 50 (7), 1118-1125